(19) Europäisches Patentamt
European Patent Office
Office européen des brevets

(11) EP 3 375 361 A1

(12) **EUROPEAN PATENT APPLICATION**
published in accordance with Art. 153(4) EPC

(43) Date of publication:
**19.09.2018 Bulletin 2018/38**

(51) Int Cl.:
*A61B 5/024* (2006.01)    *A61B 5/05* (2006.01)

(21) Application number: **16863724.7**

(22) Date of filing: **11.11.2016**

(86) International application number:
**PCT/ES2016/070804**

(87) International publication number:
**WO 2017/081353 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.11.2015 ES 201531645**

(71) Applicant: **Universitat Politècnica De Catalunya 08034 Barcelona (ES)**

(72) Inventors:
• **PALLÀS ARENY, Ramon**
  08034 Barcelona (ES)
• **CASANELLA ALONSO, Ramon**
  08034 Barcelona (ES)
• **GÓMEZ CLAPERS, Joan**
  08034 Barcelona (ES)

(74) Representative: **Juncosa Miró, Jaime et al Torner, Juncosa i Associats, S.L. Gran Via de les Corts Catalanes, 669 bis, 1o, 2a 08013 Barcelona (ES)**

(54) **METHOD AND DEVICE FOR ESTIMATING THE TRANSIT TIME OF THE ARTERIAL PULSE FROM MEASUREMENTS OBTAINED IN DISTAL ZONES OF THE EXTREMITIES**

(57)    The invention relates to a method and device for estimating the arterial pulse transit time (PTT) in different arterial sections from measurements carried out using sensors disposed only in distal zones of the two upper extremities or the two lower extremities. Firstly, the arrival of the pulse wave at a zone of the extremities next to the torso is detected in the impedance plethysmograph (IPG) measured between the two upper extremities or between the two lower extremities, which reflects changes in zones closest to the zone where the measuring electrodes are disposed. Secondly, another time reference is obtained from a pulse signal measured in a distal zone using traditional methods. When the first pulse signal is the IPG between the two upper extremities, the second pulse signal can be the IPG between the two lower extremities. The PTT is estimated from the time interval measured between the respective arrival instants of the pulse wave in the first and in the second signal.

Figure 1

## Description

## FIELD OF THE INVENTION

The present invention relates, in general, to systems for measuring physiological parameters through physical methods and, more specifically, to a method and to a device for estimating the arterial pulse transit time from measurements obtained from sensors exclusively placed at distal areas of the extremities.

## BACKGROUND OF THE INVENTION

The pulse transit time (PTT) is defined as the lapse between the arrival of the blood pulse wave to a proximal site with respect to the heart and the arrival to a distal site. Therefore, it describes the propagation of the blood pulse wave generated from cardiac ejection into the aorta and is an important parameter for assessing the health status of the cardiovascular system. The PTT allows, for instance, the assessment of the stiffness of the arteries, which is increasingly becoming a widely-accepted marker of cardiovascular disease risk. Arterial stiffness has been associated to the presence of cardiovascular risk factors and arteriosclerotic disease, and its suitability for predicting risk of future cardiovascular events such as myocardial infarction, stroke, revascularization or aortic syndromes, among others, has been widely corroborated, as described in the document by C. Vlachopoulos, K. Aznaouridis, and C. Stefanadis, "Prediction of Cardiovascular Events and All-cause Mortality With Arterial Stiffness: a Systematic Review and Meta-analysis," Journal American College Cardiology, vol. 55, no. 13, pp. 1318-27, Mar. 2010.

Another parameter closely related to the arterial elasticity is blood pressure, since the modulus of elasticity of an artery E depends on changes in mean blood pressure P according to

$$ E = E_0 e^{kP} \ , $$

where $E_0$ is the elasticity modulus of the artery at a reference mean arterial pressure and $k$ is a constant that depends on the artery and whose value is comprised between 0.016 mmHg$^{-1}$ and 0.018 mmHg$^{-1}$. Therefore, changes in arterial blood pressure and absolute values of arterial blood pressure can be estimated from PTT measurements in the aorta or in other arteries by using different calibration methods, as described, for example, in the document by D. Buxi, J. M. Redoute, and M. R. Yuce, "A Survey on Signals and Systems in Ambulatory Blood Pressure Monitoring Using Pulse Transit Time," Physiological Measurements, DOI 10.1088/0967-3334/36/3/R1.

Among the arteries of the human body, the elasticity of the aorta has the highest clinical significance since the aorta is responsible for most of the pathophysiological effects derived from arterial stiffness, and constitutes the best indicator of the overall stiffness of the arteries of a subject. Aortic stiffness has shown the highest predictivity of cardiovascular events in several epidemiologic studies, as described in the document by L. M. Van Bortel, S. Laurent, P. Boutouyrie, P. Chowienczyk, J. K. Cruickshank, et al., "Expert Consensus Document on the Measurement of Aortic Stiffness in Daily Practice Using Carotid-femoral Pulse Wave Velocity," Journal Hypertension, vol. 30, no. 3, pp. 445-448, Mar. 2012. However, the overall stiffness of the arteries of a subject can be also evaluated from other major arteries since these reflect changes with medical significance as well, as described, for instance, for the arteries of the forearm, calf, and hand in the document by I. Hlimonenko, K. Meigas, M. Viigimaa, and K. Temitski, "Assessment of Pulse Wave Velocity and Augmentation Index in Different Arteries in Patients With Severe Coronary Heart Disease," en Engineering in Medicine and Biology Society (EMBS), 2007. 29th Annual International Conference of the IEEE, Aug. 2007, pp. 1703-1706.

Arterial stiffness is normally noninvasively evaluated from the propagation speed of the blood pulse wave, the so-called pulse wave velocity (PWV), according to the Moens-Korteweg's formula,

$$ PWV = \sqrt{\frac{Eh}{2r\rho}} \ , $$

where $E$ is the elastic modulus of the artery, $h$ is the thickness of the arterial wall, $r$ is the arterial radius and $\rho$ is the blood density. Respectively, PWV in an artery can be obtained from the PTT measured between a proximal and distal site respect to the heart in said artery, according to

$$ PWV = \frac{D}{PTT} \ , $$

where $D$ is the distance between the proximal and distal sites considered.

The common procedure for measuring aortic PTT require preparation (to expose, clean, place the sensors and connect the cables) of a proximal site and a distal site with respect to the heart in order to detect the arrival of the blood pressure pulse to each of those points by means of, for instance, a photoplethysmograph (PPG) or an impedance plethysmograph (IPG) that detects local volume changes due to the arrival of the pressure pulse, or by means of an arterial tonometer that measures the pressure that a superficial artery exerts onto a force sensor in close contact to it.

Nevertheless, the placement of these and other sensors at the torso or close areas require skill in their placement, and entail slow procedures and some embarrassment for the subject because those areas are normally covered by clothing. A method to ease the measurement is to place the distal sensor at hands or feet, which is especially convenient for measurements in ambulatory scenarios because the first are commonly exposed and the latter are easily accessible. However, the distance between sensors $D$ must be long to achieve a low uncertainty in a PTT measurement and therefore the placement of a sensor at the torso or a close area to detect the arrival of the blood pressure pulse to a proximal site respect to the heart is still required, which hinders and lengthens the measurement.

An alternative method to obtain proximal information without placing sensors at the torso is to detect the R wave of the electrocardiogram (ECG), which can be obtained from electrodes placed at distal sites such as hands or feet. However, the time interval from the ECG R wave or other fiducial points to the arrival of the blood pressure pulse to a distal area, the so-called pulse arrival time (PAT), includes a fraction of the pre-ejection period (PEP), the electromechanical delay defined as the time interval between the Q wave of the ECG and the opening of the aortic valve, which marks the onset of the blood pressure pulse. The PAT can be used to measure changes in the PTT and to assess the arterial stiffness, as described in the previously cited document by D. Buxi, J. M. Redoute, and M. R. Yuce, "A Survey on Signals and Systems in Ambulatory Blood Pressure Monitoring Using Pulse Transit Time," Physiol. Meas., DOI 10.1088/0967-3334/36/3/R1, but this method is only applicable when the changes in PEP are comparatively insignificant with respect to the changes in PTT. For this reason, the use of the PAT to estimate changes in the PTT is discouraged. Patent WO 2013017718 A2 describes a method and apparatus for monitoring the cardiovascular system from time intervals measured from the ECG to the IPG measured between upper limbs or lower limbs, which is equivalent to the PAT and not to the PTT because it includes the PEP.

Another alternative method to detect the arrival of the blood pressure pulse to the torso or close areas is from certain fiducial points of the ballistocardiogram (BCG), as described in the document by R. Pallas Areny, R. Casanella, and J. Gomez-Clapers, "Metodo y aparato para estimar el tiempo de tránsito del pulso aórtico a partir de intervalos temporales medidos entre puntos fiduciales del ballistocardiogrma," P201531414. As BCG reflects changes in the center of mass of the human body resulting from mechanical activity related to cardiac ejection, in the document the use of BCG waves is proposed as a time reference for proximal and distal changes in the aorta to measure PTT between these waves. However, since BCG is acquired in subjects standing on a scale, the method and device described in said document do not cover other situations in which the subject is not standing and in which it may also be interesting to determine the moments in which cardiovascular events occur in general, and specifically to determine the arrival of the pulse wave at proximal and distal points with respect to the heart.

Another alternative method to detect the arrival of the blood pressure pulse to the torso or close areas is by measuring electrical impedance in the thorax between the neck and the abdomen, the so-called impedance cardiogram (ICG), which reflects plethysmographic changes along the entire length of the aortic artery, as described in the document by L. Jensen, J. Yakimets, and K. K. Teo, "A Review of Impedance Cardiography," Hear. Lung J. Acute Crit. Care, vol. 24, no. 3, pp. 183-193, May 1995. However, the ICG requires the injection of an electric current that circulates along the torso, and this is usually achieved by placing electrodes on the neck and abdomen, which makes it unsuitable for rapid measurements or outside hospital environments, and even more so when it is not only the elasticity of the aorta that is to be evaluated but also that of the arteries of the upper or lower extremities.

In patent US6228033 B1 a system to monitor the cardiovascular system called whole-body ICG analogous to the ICG is described, in which the measurement of thoracic impedance is performed by injecting current from a first terminal connected to one or two upper limbs, and a second terminal connected to one or two lower limbs, so that it does not require any exposure of the torso. However, the method always requires the exposure of at least one upper limb and at least one lower limb hence it is not well suited to elasticity measurements of arteries exclusively at the upper limbs or exclusively at the lower limbs.

Patent WO 2012103296 A2 describes a device and method for monitoring the cardiovascular system in which the interval between a signal reflecting the movement of blood in the aorta from the ICG measured between the upper extremities or between the lower extremities and a photoplethysmographic sensor located in a distal area is measured. However, measuring plethysmographic variations in the aorta from an impedance signal measured between extremities is complicated, since the contribution of these variations to the measured waveform is very small as compared to the contributions of other arteries in the extremities, so the uncertainty in the value of the measured interval is expected to be large.

The use of the IPG measured between the two upper extremities or between the two lower extremities to detect plethysmographic changes in areas proximal to the torso, corresponding to the extremities rather than being associated with changes in the aorta, combined with another pulse sensor located in a distal area of the upper or lower extremities, would allow PTT to be measured in different arterial segments more quickly, comfortably and reliably than with the current methods and systems, already in use. This would avoid the placement of sensors in areas proximal to the torso, which would be very useful for evaluating the elasticity of the arteries and its derived parameters.

## SUMMARY OF THE INVENTION

The invention consists of a method and device for estimating the arterial pulse transit time (PTT) from measurements obtained by means of sensors arranged exclusively in distal areas of the extremities.

The innovative solution proposed by the present invention is the use of the impedance plethysmograph signal measured between the two upper extremities or between the two lower extremities, i.e. along the left-right axis of the human body, to detect plethysmographic changes in areas closer to the torso, corresponding to the proximal part of the extremities, than those areas where the sensors are placed, which in this case are the electrodes that obtain IPG. This allows the PTT to be measured in an arterial segment from the time interval between the plethysmographic signal "from one side to the other side" and another signal provided by a second pulse wave sensor placed in a distal area of the extremities, either from the local IPG, i.e. with electrodes around a small area of the extremity, the PPG, a tonometer, or another cardiovascular event sensor, such as the BCG. Since both the signal from the second sensor and the signal from the IPG in the proposed method are obtained by measurements using sensors arranged either in the distal parts of the upper extremities or the lower extremities, or placed on a support with which the upper extremities or the lower extremities make contact, the PTT can be measured without the need to place pulse sensors in areas proximal to the torso and this allows for quick, convenient and even autonomous measurement when the same measured person comes into contact with the electrodes, rather than having them placed on the extremities by another person.

This innovative solution is based on the fact that the IPG signal measured between the two upper extremities or between the two lower extremities reflects plethysmographic changes along the path followed by the injected current. Since the current flows from one side of the body to the other side through the torso, when measuring between the two upper extremities, it is expected that the waveform of this IPG will correspond to the superimposition of plethysmographic changes in the path of the current caused by the arrival of the pulse wave to the different arteries of the upper thorax and upper extremities. However, the contribution of plethysmographic changes in the aorta or torso to the waveform obtained by the IPG measured between extremities is very small, given that the arteries with a larger diameter have a lower impedance, which makes them difficult to detect with this IPG and their measurement is unreliable. Therefore, the solution proposed in this invention is to detect the arrival of the pulse wave to parts of the upper extremities proximal to the torso, since its contribution to the waveform is much greater and therefore more easily detectable. Similarly, the waveform of the IPG measured between the two lower extremities is expected to correspond to the overlap of plethysmographic changes in the path of current caused by the arrival of the pulse wave to the different arteries of the lower abdomen and lower extremities. Since the contribution of the aortic and iliac arteries to the obtained waveform is very low due to their larger diameter, which implies lower impedance, it is proposed to detect the arrival of the pulse wave to parts of the lower extremities proximal to the torso, as its contribution to the waveform is much greater and therefore more easily detectable.

A particular way to measure the PTT from the impedance signal between extremities is by using the BCG. Since the BCG signal obtained, for instance, from the sensors of a scale on which a person stands provides time information related to the heart ejection in its initial waves, such as the I wave, and time information related to the arrival of the pulse wave at the end of the aorta, such as the J wave, as detailed in document P201531414, such waves I and J can be used in combination with IPG measurements between, respectively, the two lower extremities or the two upper extremities to obtain a PTT that would include the aortic PTT and the upper leg or arm PTT, respectively.

As a result, a method to estimate the PTT in a section of the arterial tree is proposed, consisting, first, of detecting a fiducial point of the pulse wave in the IPG signal measured between the two upper extremities or between the two lower extremities, corresponding to the arrival of the pulse wave to an area proximal to the thorax near those extremities, and a fiducial point of a second signal obtained from a pulse sensor placed in a distal area of a extremity such as a finger or toe, or on a scale in contact with the two lower extremities of a person standing on it. When the first signal is the IPG between the two upper extremities, the second pulse signal may be the IPG between the two lower extremities. Then, in any case, the time interval between the fiducial point of the first IPG signal and the fiducial point of the second pulse signal is measured, and this interval corresponds to the PTT in a certain segment of the arterial network.

Several of the algorithms commonly used to automatically detect the onset of the pulse wave in a heartbeat can be used to detect plethysmographic changes proximal to the torso in the IPG signal measured between the two upper extremities or between the two lower extremities, for example, the detection of its minimum value, a threshold value of the wave's ascending impulse (10%, 25%, 50%, etc.), the maximum of the first derivative, the maximum of the second derivative, or the intersection of tangent lines, among others, as detailed for example in the document of X. Zhou, R. Peng, H. Ding, N. Zhang, and P. Li, "Validation of New and Existing Decision Rules for the Estimation of Beat-to-Beat Pulse Transit Time," Biomed Res. Int., vol. 2015, Article ID 306934, pp. 1-13, Mar. 2015. The influence of plethysmographic changes proximal to the torso on the obtained waveform allows these to be detected with any traditional algorithm based on the detection of the arrival of the pulse wave on local plethysmographic signals, unlike plethysmographic changes in the torso that require specific algorithms and are less reliable if applied to the IPG signal measured between the extremities.

An optimal implementation of the proposed method would be by means of a device comprising: a set of electrodes and other sensors integrated in the body of the device suitable for contact by the subject, either by touching, grasping or holding, arranged in such a way that it is possible to obtain from them the IPG between the two upper extremities or between the two lower extremities; an IPG system connected to these electrodes; another system integrated into the device which obtained a cardiac pulse signal from a second sensor placed in a distal area of these upper or lower extremities; the signal processing systems necessary to automatically detect the arrival of the pulse wave to a proximal torso area in the IPG signal measured between extremities and the arrival of the pulse wave to the second sensor in a distal area; the computation systems required to calculate the time interval between these two fiducial points; and which finally contained a communication system for the PTT obtained which would be responsible for its representation in a display element or for the communication of the measured value to another apparatus.

The electrodes of the proposed device could be easily integrated, for example, into a mobile phone housing, into a bar of an exercise apparatus or into a device for measuring other body parameters, such as weight or body composition by bioimpedance analysis, where the bars mentioned above are grasped with the hands. In the case of the lower extremities, the electrodes could be easily integrated into devices on which the feet rest, such as scales or other mechanical platforms. In all the above examples, the proposed device would allow a fast, comfortable, autonomous and non-invasive measurement of the elastic properties of the arteries detected by the proposed method.

The main advantage of the invention described here is that it allows the arterial PTT to be obtained by measuring only in distal areas of the extremities, which makes it possible to measure the PTT more quickly, comfortably, autonomously and reliably than systems that require the placement of at least one sensor in a proximal area to the heart.

**BRIEF DESCRIPTION OF THE DRAWINGS**

To complement the description, and to allow a better comprehension of the features of the present invention, the following illustrative, exemplary, representative, and non-limiting figures, which form an integral part of this specification, are also provided, in which:

Figure 1 - Shows the diagram of a system able of obtaining the IPG between the hands and also a distal plethysmographic signal (PPG), which constitutes the element with which the subject comes into contact in one of the embodiments of the present invention.

Figure 2 - Shows the typical waveform of the IPG measured between one hand and the other hand together with the local plethysmographic (PPG) signals measured on the shoulder, elbow, wrist, and index finger of the same extremity.

Figure 3 - Shows the respective path of the current injected through the body in an IPG measurement between one hand and the other hand ($i_{IPGh}$), in an IPG measurement between one foot and the other foot ($i_{IPGf}$), and in an ICG measurement ($i_{ICG}$), the latter obtained by injecting current between electrodes placed on the neck and abdomen according to the usual procedure for obtaining the ICG.

Figure 4 - Shows the linear regression analysis and the Bland-Altman analysis of 480 pairs of simultaneous PTT measurements obtained with the proposed method and the PTT in the carotid-index finger segment, measured with a conventional method, i.e., between the signal of one pulse sensor in the proximal area and that of another pulse sensor in the distal area.

Figure 5 - Shows the diagram of a system able of obtaining the IPG between one foot the other foot, and also the local IPG in one foot, and which constitutes the element with which the subject comes into contact in another embodiment of the present invention.

Figure 6 - Shows the typical waveform of the IPG measured between one foot and the other foot together with the local plethysmographic signals (PPG) measured at the beginning of the femoral artery, knee and ankle of the same extremity.

Figure 7 - Shows the diagram of a system able of obtaining the IPG between one foot and the other foot, and also a BCG, and which constitutes the element with which the subject comes into contact in another embodiment of the present invention.

**DETAILED DESCRIPTION OF VARIOUS EMBODIMENTS**

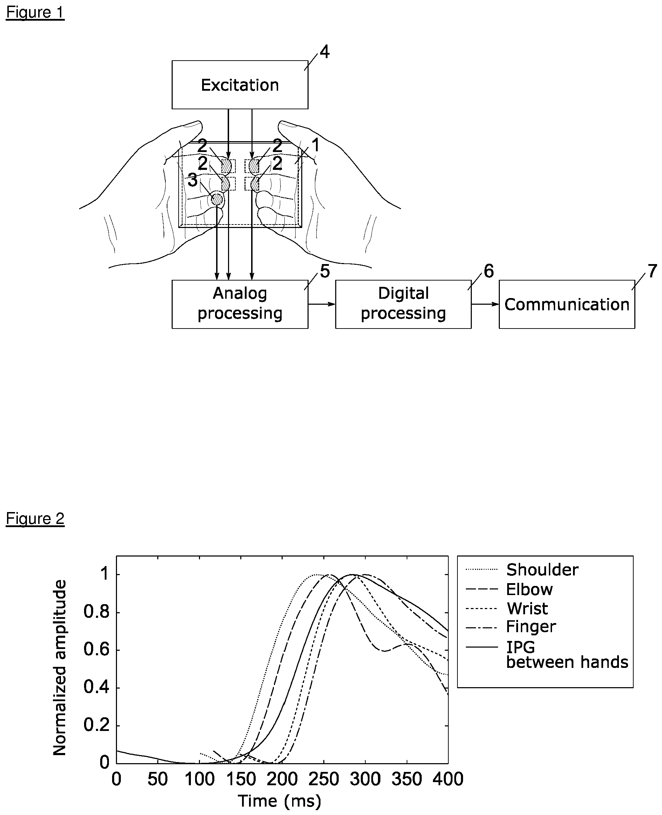

In a first embodiment of the invention, shown in figure 1, the PTT in an arm is measured from a system integrated in a hand device (1) consisting of two pairs of electrodes (2) in contact with the index and middle fingers of each hand of the subject, and a PPG sensor (3) in contact with the ring finger of the hand of the arm to be examined.

The IPG signal between the two upper extremities is obtained from an excitation system (4), which injects a high frequency current circulating from the index finger of one hand to the index finger of the other hand through the upper extremities and the upper part of the subject's thorax, and from an analog processing system (5), which measures, between the middle finger of one hand and the middle finger of the other hand.

A digital processing module (6) then detects the foot of the IPG wave measured between the two hands, and the foot of the PPG wave measured on the ring finger, and calculates the time difference between the two points, which corresponds to the PTT on the subject's arm. Finally, the communication module (7) communicates the estimated PTT value of the subject through an LCD monitor.

Figure 2 shows the IPG measured between the hands and different local plethysmographic waves measured simultaneously and obtained with a PPG sensor successively located on the shoulder, elbow, wrist and ring finger. It can be observed how, although the beginning of the ascent of the IPG wave measured between the hands is anterior to that of the other the pulse waves obtained with the PPG, the section of maximum slope is posterior to the arrival of the pulse wave to the shoulder and elbow, so it follows that the traditional algorithms, based on the detection of this segment, when applied to the IPG measured between the two hands, detect plethysmographic changes in areas of the extremities proximal to the torso and not in the aorta or torso.

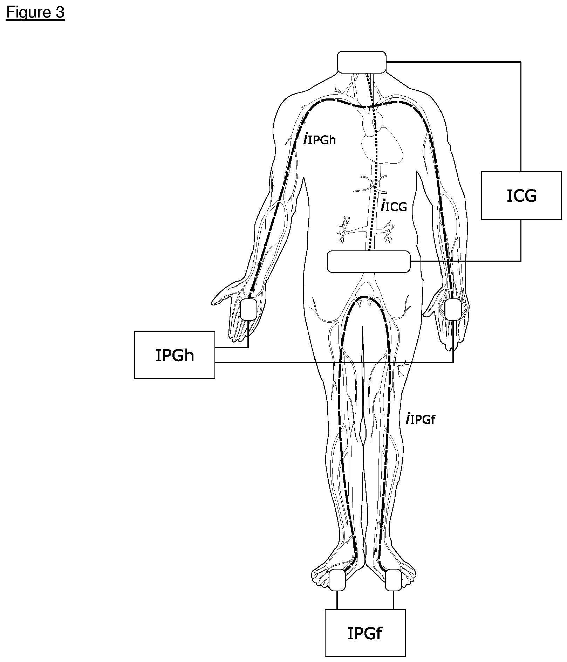

To better illustrate the difference between the IPG measured between one hand and the other hand, IPGh, the IPG measured between one foot and the other foot, IPGf, and the ICG measured between the neck and the abdomen according to the usual procedure, Figure 3 shows in simplified form the respective path of the current injected by each system. Although the path of the current injected by the IPG measured between one hand and the other hand $i_{IPGh}$ and the path of the current injected by the $i_{ICG}$ coincide in the aortic arch, the rest of the two paths are completely different so that the waveform obtained by each system will be determined by the particularities of the respective path and should not show any other coincidence in principle. Similarly, the path of the current injected by the IPG measured between one foot and the other foot $i_{IPGf}$ and the path of the current injected by the ICG $i_{ICG}$ coincide only in the abdomen area, so the nature of the two signals is expected to be completely different except at that point.

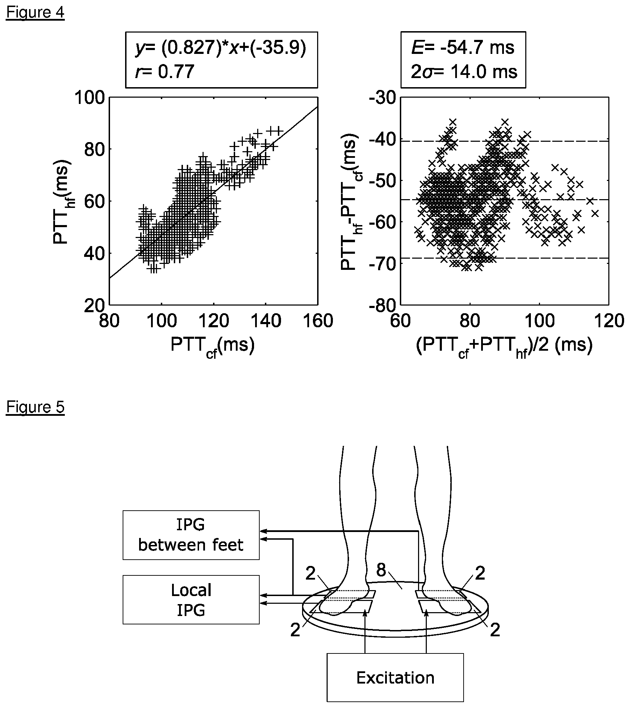

To illustrate the correspondence between the PTT obtained with the proposed method and other PTT obtained with standard methods, the PTT hand-to-hand finger obtained with the proposed method (PTThf) and the PTT in the segment between the carotid artery and the index finger measured with two tonometers placed on the carotid finger (PTTcf) were simultaneously measured. The arrival of the pulse wave was detected with the slope intersection method in both cases. Figure 4 shows the linear regression analysis and the Bland-Altman analysis of 480 pairs of simultaneous PTT measurements, obtained in several subjects under rhythmic breathing in order to induce changes in PTT; the graphs show a good agreement between the values of both parameters and it can be seen how the pulse wave arrival at the IPG measured between the upper extremities is 54.7 ms after the arrival of the pulse wave to the carotid artery, so it follows that the plethysmographic changes detected in the IPG signal measured between the two upper extremities correspond to an area of these extremities and not to changes in the aorta or torso.

In another preferred embodiment of the invention, analogous to the previous one and shown in figure 5, the PTT in one leg is measured from a system integrated in a domestic weight scale (8) consisting of two pairs of electrodes (2), one electrode in contact with the front part of the foot sole and another electrode in contact with the heel of each foot of the subject, and from which the IPG is obtained between the two extremities.

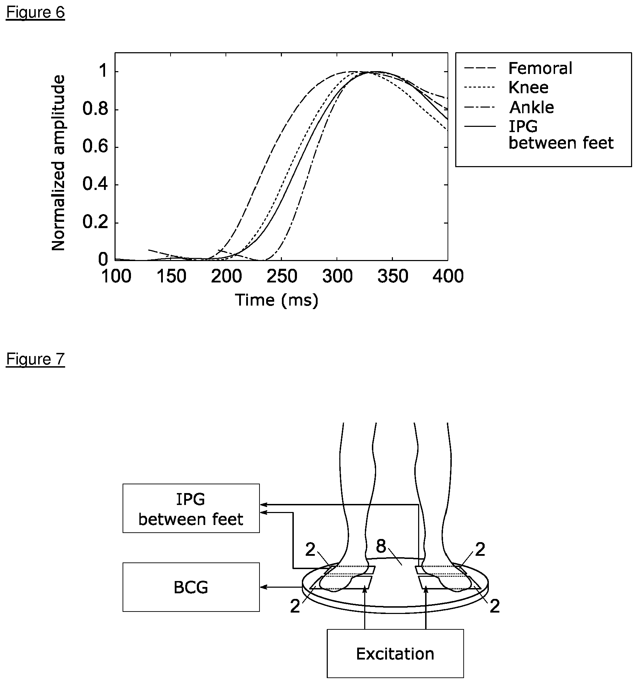

Figure 6 shows the IPG measured between the two lower extremities and different local plethysmographic waves simultaneously measured and obtained with a PPG sensor successively located at the hip, knee and ankle. It can be observed how, although the beginning of the ascent of the IPG wave measured between the feet is simultaneous with the pulse wave obtained with the PPG at the femoral point, the maximum slope is subsequent to the arrival of the pulse wave at the femoral point and the knee, so it follows that the traditional algorithms, based on the detection of this segment, when applied to the IPG measured between the two feet, detect plethysmographic changes in areas of the lower extremities proximal to the torso and not changes in the aorta or torso.

Thus, although the method proposed in this invention cannot measure plethysmographic changes produced exclusively in the aorta or torso, it offers the advantage of allowing the use of algorithms of proven reliability that allow the robust detection of the arrival of the pulse wave at points proximal to the torso, which may be used to obtain PTT in arteries that are also of interest for a comfortable and non-invasive monitoring of the properties of the circulatory system.

In another preferred embodiment of the invention, shown in Figure 7, the PTT in the aorta is measured from a system integrated in a domestic weight scale (8) comprising two pairs of electrodes (2), an electrode in contact with the front of the sole of the foot and another electrode in contact with the heel of each foot of the subject, from which the IPG is obtained between the feet by injecting current between the two feet and measuring the voltage between them; and a system for obtaining the ballistocardiogram (BCG) from one or more force sensors integrated in the platform. Since the initial BCG waves, such as wave I, reflect the heart ejection, the delay between these waves and the arrival time of the pulse wave from the IPG to the lower extremities corresponds to the PTT in the aorta and a section of the femoral artery.

Once the invention has been sufficiently described, it should only be added that it is possible to make modifications in its constitution, materials used, and in the choice of the sensors used to obtain the second distal signal, and in the methods to identify the arrival of the pulse wave in this signal and in the IPG measured between the two upper extremities or between the two lower extremities, without deviating from the scope of the invention, defined in the following claims.

**Claims**

1.  A method for estimating pulse transit time (PTT) from measurements obtained exclusively in distal areas of the extremities **characterized in that**

    a) a high frequency current or voltage is injected between the two upper extremities or between the two lower extremities so that at least one electrode is located in a distal area of each of the two extremities concerned;
    b) the voltage is measured at the frequency injected between at least one electrode in a distal area of each of these two extremities;
    c) the impedance plethysmograph (IPG) between these two extremities is extracted from the pulsed component of the voltage drop;
    d) the arrival of the pulse wave to an area of the two extremities proximal to the torso is detected from the start of the pulse wave of the IPG measured between these two extremities for each heartbeat;
    e) a fiducial point on a second pulse wave of the same beat and corresponding to the arrival of the pulse wave to another area is detected by a sensor placed in a distal area of the upper or lower extremities;
    f) the time interval between the start of the pulse wave of the IPG measured between the two extremities and the fiducial point of the second pulse wave for each beat is measured;
    g) a PTT is obtained from the time interval measured between the pulse waves in the two signals obtained.

2.  A method according to claim 1, **characterized in that** the second pulse wave is obtained from a local IPG.

3.  A method according to claim 1, **characterized in that** the second pulse wave is obtained from a photoplethysmogram (PPG).

4.  A method according to claim 1, **characterized in that** the second pulse wave is obtained from an arterial tonometer.

5.  A method according to claim 1, **characterized in that** the first pulse wave is an IPG measured between the two hands and the second pulse wave is an IPG measured between the two feet.

6.  A method according to claim 1, **characterized in that** the second pulse wave is obtained from a ballistocardiogram (BCG).

7.  A method according to claims 1 to 4, **characterized in that** the PTT in one arm is estimated from the beginning of the pulse wave of the IPG measured between the two hands and the beginning of the pulse wave of a plethysmographic sensor such as that of claims 2, 3 or 4 placed in the hand of that arm.

8.  A method according to claims 1 to 4, **characterized in that** the PTT in one leg is estimated from the beginning of the pulse wave of the IPG measured between the two feet and the beginning of the pulse wave of a plethysmographic sensor such as that of claims 2, 3 or 4 placed on the foot of that leg.

9.  A method according to claims 1 to 4, **characterized in that** the PTT in the aorta and one leg is estimated from the beginning of the pulse wave of the IPG measured between the two hands and the beginning of the pulse wave of a plethysmographic sensor such as that of claims 2, 3 or 4 placed on the foot of that leg.

10. A method according to claims 1 and 5, **characterized in that** the PTT in the aorta and part of the arms and legs is estimated from the beginning of the IPG wave measured between the two hands and the beginning of the IPG wave measured between the two feet.

11. A method according to claims 1 and 6, **characterized in that** the PTT in the aorta and part of the legs is estimated from BCG wave I and the beginning of the pulse wave in the measured IPG between the two feet.

12. A method according to claims 1 and 6, **characterized in that** the PTT in the aorta and part of the arms is estimated from the beginning of the pulse wave of the IPG measured between the two hands and the J wave of the BCG.

13. A device capable of automatically estimating the PTT in an arterial segment from plethysmographic measurements obtained exclusively in distal areas of the extremities, comprising:

    a) a set of electrodes integrated into the surface of the device configured for being contacted by the subject, either by touching, grasping or holding them, arranged in such a way that it is possible to obtain from them the IPG between the two upper extremities or between the two lower extremities;

b) an IPG measurement system connected to said electrodes;

c) a system which obtains a second cardiac pulse signal from a sensor suitable for placement in a distal area of the upper or lower extremities;

d) a signal processing system configured to automatically detect the time of arrival of the pulse wave in a zone proximal to the torso from the IPG measured between the extremities and in another zone from the second pulse signal;

e) a calculation system configured to obtain the time interval between the respective instants of arrival of the pulse wave detected with the system in the previous paragraph;

f) a calculation system configured to obtain the PTT from said time interval;

g) a communication system configured to communicate the calculated PTT to a user or other apparatus.

14. A device according to claim 13 **characterized in that** the electrode array is integrated into the housing of a mobile phone, tablet or remote control of a television or other household appliance.

15. A device according to claim 13 **characterized in that** the set of electrodes is integrated into an exercise device handlebar.

16. A device according to claim 13 **characterized in that** set of electrodes is integrated into a handlebar of a device measuring body parameters such as weight or body composition.

17. A device according to claim 13 **characterized in that** the set of electrodes is integrated in a scale.

18. A device according to claim 13 and 14, 15, 16 or 17 **characterized in that** the system obtaining a cardiac pulse signal from a sensor placed in a distal area of the upper or lower extremities is an impedance plethysmograph which detects local volume changes in the area near the voltage measuring electrodes.

19. A device according to claim 13 and 14, 15, 16 or 17 **characterized in that** the system obtaining a cardiac pulse signal from a sensor placed in a distal area of the upper or lower extremities is a photoplethysmograph.

20. A device according to claim 13 and 14, 15, 16 or 17 **characterized in that** the system obtaining a cardiac pulse signal from a sensor placed in a distal area of the upper or lower extremities is an arterial tonometer.

21. A device according to claim 13 and 14, 15, 16 or 17 **characterized in that** the system obtaining a cardiac pulse signal from a sensor placed in a distal area of the upper or lower extremities uses the same electrodes as the impedance plethysmograph obtaining the IPG between the extremities.

22. A device according to claims 13 and 14, 15, 16 or 17 **characterized in that** the system obtaining a cardiac pulse signal from a sensor placed in a distal area of the upper or lower extremities is a ballistocardiograph in contact with the feet.

23. A device according to claim 13, 18 and 14, 15, 16 or 17 **characterized in that** the impedance plethysmograph that detects local volume changes in the area near to that where the voltage measuring electrodes are located, uses in a shared way the electrodes of the system obtained by the IPG between the extremities that are located in that of the two extremities where the local IPG is to be detected.

Figure 1

Figure 2

Figure 3

Figure 4

$y= (0.827)*x+(-35.9)$
$r= 0.77$

$E= -54.7$ ms
$2\sigma= 14.0$ ms

Figure 5

Figure 6

Figure 7

## INTERNATIONAL SEARCH REPORT

| International application No. |
| --- |
| PCT/ES2016/070804 |

**A. CLASSIFICATION OF SUBJECT MATTER**

*A61B5/024* (2006.01)
*A61B5/05* (2006.01)
According to International Patent Classification (IPC) or to both national classification and IPC

**B. FIELDS SEARCHED**

Minimum documentation searched (classification system followed by classification symbols)
A61B

Documentation searched other than minimum documentation to the extent that such documents are included in the fields searched

Electronic data base consulted during the international search (name of data base and, where practicable, search terms used)

EPODOC, INVENES, WPI

**C. DOCUMENTS CONSIDERED TO BE RELEVANT**

| Category* | Citation of document, with indication, where appropriate, of the relevant passages | Relevant to claim No. |
| --- | --- | --- |
| X | WO 2012103296 A2 (UNIV LELAND STANFORD JUNIOR ET AL.) 02/08/2012, The whole document, especially p.3 l.17-26; p. 5, l. 25-27; p. 8, l.30 – p.9, l.8; p. 13, l.25 – p.14, l.5; p. 15, l. 11-18; p. 16, l.28 – p. 17, l.8; p. 35, l.12–22; Figs. 4, 14A | 1, 3, 4, 6, 11 - 17, 19, 20, 22 |
| A | | 2, 5, 7 – 10, 18, 21, 23 |
| A | EP 2737847 A2 (UNI POLITÈCNICA DE CATALUNYA) 04/06/2014, The whole document, especially [0004], [0013], [0020] Fig. 4 | 1 - 23 |
| A | EP 2074942 A1 (SUISSE ELECTRONIQUE MICROTECH) 01/07/2009, The whole document, especially [0057], [0060], [0076] | 1 - 23 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
| --- | --- |
| 23/12/2016 | **(04/01/2017)** |
| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> J. Carbonell Olivares <br><br><br> Telephone No. 91 3496837 |

Form PCT/ISA/210 (second sheet) (January 2015)

## INTERNATIONAL SEARCH REPORT

| International application No. |
|---|
| PCT/ES2016/070804 |

**C (continuation).** **DOCUMENTS CONSIDERED TO BE RELEVANT**

| Category * | Citation of documents, with indication, where appropriate, of the relevant passages | Relevant to claim No. |
|---|---|---|
| A | WO 2008004159 A2 (PHILIPS INTELLECTUAL PROPERTY ET AL.) 10/01/2008, The whole document, especially p. 4, l. 7 -20; Figs. 2, 7 | 1 - 23 |
| A | EP 1344489 A1 (GE MED SYS INFORMATION TECH) 17/09/2003, The whole document, especially Figs. 4, 7 | 1 - 23 |

Form PCT/ISA/210 (continuation of second sheet) (January 2015)

| INTERNATIONAL SEARCH REPORT | | International application No. | |
|---|---|---|---|
| Information on patent family members | | PCT/ES2016/070804 | |
| Patent document cited in the search report | Publication date | Patent family member(s) | Publication date |
| WO2012103296 A2 | 02.08.2012 | JP2016101504 A | 02.06.2016 |
| | | US2016095522 A1 | 07.04.2016 |
| | | US2015282718 A1 | 08.10.2015 |
| | | US9241637 B2 | 26.01.2016 |
| | | JP2014507213 A | 27.03.2014 |
| | | JP5955341B B2 | 20.07.2016 |
| | | US2013310700 A1 | 21.11.2013 |
| | | US9011346 B2 | 21.04.2015 |
| | | CA2825405 A1 | 02.08.2012 |
| | | AU2012211300 A1 | 09.05.2013 |
| | | EP2667769 A2 | 04.12.2013 |
| | | EP2667769 A4 | 22.10.2014 |
| EP2737847 A2 | 04.06.2014 | JP2014521427 A | 28.08.2014 |
| | | US2014194721 A1 | 10.07.2014 |
| | | US9510761 B2 | 06.12.2016 |
| | | KR20140065395 A | 29.05.2014 |
| | | CN103826531 A | 28.05.2014 |
| | | CN103826531B B | 12.10.2016 |
| | | ES2398439 A2 | 19.03.2013 |
| | | ES2398439 B1 | 05.03.2014 |
| | | WO2013017718 A2 | 07.02.2013 |
| | | WO2013017718 A3 | 28.03.2013 |
| EP2074942 A1 | 01.07.2009 | AT543430T T | 15.02.2012 |
| | | US2009163821 A1 | 25.06.2009 |
| | | US8585605 B2 | 19.11.2013 |
| WO2008004159 A2 | 10.01.2008 | JP2009542294 A | 03.12.2009 |
| | | US2009204013 A1 | 13.08.2009 |
| | | US8233969 B2 | 31.07.2012 |
| | | CN101484068 A | 15.07.2009 |
| | | EP2040616 A2 | 01.04.2009 |
| | | EP2040616 B1 | 03.10.2012 |
| EP1344489 A1 | 17.09.2003 | US2003167012 A1 | 04.09.2003 |
| | | US6648828 B2 | 18.11.2003 |
| | | JP2003265421 A | 24.09.2003 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013017718 A2
- US 6228033 B1
- WO 2012103296 A2


**Non-patent literature cited in the description**

- **C. VLACHOPOULOS ; K. AZNAOURIDIS ; C. STEFANADIS.** Prediction of Cardiovascular Events and All-cause Mortality With Arterial Stiffness: a Systematic Review and Meta-analysis. *Journal American College Cardiology,* March 2010, vol. 55 (13), 1318-27
- **D. BUXI ; J. M. REDOUTE ; M. R. YUCE.** A Survey on Signals and Systems in Ambulatory Blood Pressure Monitoring Using Pulse Transit Time. *Physiological Measurements*
- **L. M. VAN BORTEL ; S. LAURENT ; P. BOUTOUYRIE ; P. CHOWIENCZYK ; J. K. CRUICKSHANK et al.** Expert Consensus Document on the Measurement of Aortic Stiffness in Daily Practice Using Carotid-femoral Pulse Wave Velocity. *Journal Hypertension,* March 2012, vol. 30 (3), 445-448

- **I. HLIMONENKO ; K. MEIGAS ; M. VIIGIMAA ; K. TEMITSKI.** Assessment of Pulse Wave Velocity and Augmentation Index in Different Arteries in Patients With Severe Coronary Heart Disease. *Engineering in Medicine and Biology Society (EMBS), 2007. 29th Annual International Conference of the IEEE,* August 2007, 1703-1706
- **D. BUXI ; J. M. REDOUTE ; M. R. YUCE.** A Survey on Signals and Systems in Ambulatory Blood Pressure Monitoring Using Pulse Transit Time. *Physiol. Meas.*
- **R. PALLAS ARENY ; R. CASANELLA ; J. GOMEZ-CLAPERS.** *Metodo y aparato para estimar el tiempo de tránsito del pulso aórtico a partir de intervalos temporales medidos entre puntos fiduciales del ballistocardiogrma*
- **L. JENSEN ; J. YAKIMETS ; K. K. TEO.** A Review of Impedance Cardiography. *Hear. Lung J. Acute Crit. Care,* May 1995, vol. 24 (3), 183-193
- **X. ZHOU ; R. PENG ; H. DING ; N. ZHANG ; P. LI.** Validation of New and Existing Decision Rules for the Estimation of Beat-to-Beat Pulse Transit Time. *Biomed Res. Int.,* March 2015, vol. 2015, 1-13